(19) Europäisches Patentamt
European Patent Office
Office européen des brevets

(11) Veröffentlichungsnummer: **0 325 970 B1**

(12) # EUROPÄISCHE PATENTSCHRIFT

(45) Veröffentlichungstag der Patentschrift: **07.04.93**

(51) Int. Cl.$^5$: **C07H 21/00**

(21) Anmeldenummer: **89100611.6**

(22) Anmeldetag: **14.01.89**

(54) **Träger zur enzymatischen oder chemischen und enzymatischen Umsetzung von Nukleinsäuren oder Nukleinsäurefragmenten an Festphasen.**

(30) Priorität: **23.01.88 DE 3801987**

(43) Veröffentlichungstag der Anmeldung:
**02.08.89 Patentblatt 89/31**

(45) Bekanntmachung des Hinweises auf die Patenterteilung:
**07.04.93 Patentblatt 93/14**

(84) Benannte Vertragsstaaten:
**AT BE CH DE ES FR GB GR IT LI LU NL SE**

(56) Entgegenhaltungen:
**EP-A- 0 035 719**
**EP-A- 0 090 789**

**CHEMICAL ABSTRACTS, Band 107, 1987, Seite 192, Zusammenfassung Nr. 148577s, Columbus, Ohio, US; Y. HOSTOMSKY et al.: "Solid-phase assembly of DNA duplexes from synthetic oligonucleotides", & NUCLEIC ACIDS SYMP. SER. 1987, 18(SYMP. CHEM. NUCLEIC ACID COMPON., 7TH, 1987), 241-4**

(73) Patentinhaber: **BOEHRINGER MANNHEIM GMBH**
**Sandhofer Strasse 116**
**W-6800 Mannheim 31(DE)**

(72) Erfinder: **Seliger, Heinz Hartmut, Prof. Dr.**
**Hasenweg 1**
**W-7915 Elchingen-Thalfingen(DE)**
Erfinder: **Groeger, Gabriele**
**Elchinger Strasse 6**
**W-7915 Elchingen 1(DE)**

**EP 0 325 970 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft unlösliche, polymere Träger zur enzymatischen oder chemischen und enzymatischen Umsetzung von Nukleinsäuren oder Nukleinsäurefragmenten an Festphasen.

Für die gezielte Synthese von Nukleinsäuren werden im allgemeinen Nukleinsäurefragmente, d. h. Bruchstücke ganzer Nukleinsäuren, Oligomere bzw. Monomere mittels einer Kombination chemischer und enzymatischer Reaktionen miteinander verknüpft. So können z. B. zum Aufbau von Deoxyribonukleinsäure (DNA) Oligodeoxyribonukleotidfragmente von ca. 15 - 60 Einheiten Kettenlänge chemisch synthetisiert und diese dann nach Phosphorylierung am 5′-Ende gemischt und in enzymkatalysierter Reaktion mittels DNA-Ligase zu doppelsträngiger Nukleinsäure zusammengefügt werden, wobei die richtige Positionierung der einzelnen Fragmente durch gegenseitige Watson-Crick-Basenpaarung aufgrund der Hybridisierung und somit zumindest teilweise Überlappung der Fragmente erreicht wird. Chemisch synthetisierte oder in enzymkatalysierter Reaktion gewonnene Oligoribonukleotide lassen sich entsprechend mit RNA-Ligase zu Ribonukleinsäure (RNA) verknüpfen. Analog können auch Nukleinsäuren, die sowohl DNA- als auch RNA-Fragmente enthalten als Einzelstrang erhalten werden.

Im folgenden werden unter Nukleinsäuren alle möglichen Arten und Formen von Nukleinsäuren verstanden. Es können sowohl Deoxyribonukleinsäuren als auch Ribonukleinsäuren und auch gemischte Polynukleotide sein, die sowohl aus Monomeren von Deoxyribonukleinsäuren und aus Monomeren von Ribonukleinsäuren bestehen. Weiter können die Nukleinsäuren als Einzel-oder Doppelstrang vorliegen.

Teile oder Fragmente von Nukleinsäuren können sowohl Monomere, wie Nukleoside oder Nukleotide, sein als auch Oligomere aus 2 - 100 Monomereinheiten, und auch größere Polymere mit mehreren hundert Monomereinheiten sind möglich.

Für die enzymkatalysierte Verknüpfung von Nukleinsäurefragmenten hat es sich als vorteilhaft erwiesen, wenn eines der Fragmente an einen polymeren unlöslichen Träger gebunden ist. So berichteten Cozzarelli et al. in Biochem. Biophys. Res. Comm. 28, 578 - 586 (1967) über die Verknüpfung von DNA-Fragmenten mittels einer DNA-Ligase, wobei eines der Fragmente an Cellulose gebunden ist.

T. M. Jovin & A. Kornberg beschrieben in J. Biol. Chem. 243, 250 -259 (1968) den Einsatz eines an Cellulose-Partikel gebundenen Oligomers von Deoxyribothymidin als Primer und Matrize für DNA-Polymerase.

U. Bertazzoni et al., Biochim. Biophys. Acta 240, 515 - 521 (1971) berichteten über die Kettenverlängerung von kovalent an Cellulose gebundenen Oligodeoxyribonukleotiden mittels terminaler Deoxyribonukleotidyl-Transferase.

A. Panet & H. G. Khorana, J. Biol. Chem. 249, 5213 - 5221 (1974) banden Polydeoxyribothymidin an Cellulose und verlängerten diese trägergebundene Nukleinsäure mittels Ligase um ein weiteres DNA-Fragment. Mit dem so dargestellten cellulosegebundenen Polynukleotid und einem kurzen Primer wurde mit DNA-Polymerase ein Teil des Polynukleotids repliziert.

Die Vorteile des Einsatzes immobilisierter Nukleinsäurefragmente für den enzymatischen Nukleinsäureaufbau sowie ganz allgemein für deren enzymatische Umsetzung werden vor allem in der leichten Abtrennung des immobilisierten Reaktionsprodukts von den übrigen für die Reaktion benötigten und während der Reaktion entstandenen Stoffe gesehen. Aufgrund dieser einfachen Möglichkeit zur Isolierung eines gewünschten Reaktionsprodukts kann auch durch Anwendung von Überschüssen an Enzym und/oder nicht immobilisiertem Substrat eine Verschiebung des Gleichgewichts zu höheren Ausbeuten an immobilisiertem Reaktionsprodukt, z. B. an kettenverlängertem Produkt, erreicht werden, ohne die anschließende Aufreinigung dadurch wesentlich zu erschweren.

Während Gele, wie quellbare unlösliche Polysaccharide, geeignete Träger für die enzymkatalysierte Umsetzung immobilisierter Nukleinsäuren oder Nukleinsäurefragmente sind, hat es sich aber gezeigt, daß sich solche Materialien nur schlecht eignen, um Nukleinsäuren mit chemischen Methoden an polymeren unlöslichen Trägern aufzubauen oder zu verändern.

So müssen z. B. bei der chemischen Synthese von Nukleinsäuren, die üblicherweise von entsprechenden Monomeren, wie Nukleosiden oder Nukleotiden ausgeht, üblicherweise Zyklen durchlaufen werden, die im wesentlichen aus den Schritten: Aktivierung des zu verlängernden Nukleinsäureteils, eventuelle Isolierung des Produkts, Zugabe und Ankondensation eines neuen Nukleosids oder Nukleotids und Isolierung des Produkts bestehen.

Die teilweise oder vollständige Automatisierung solcher chemischer Verfahren zum Aufbau von Nukleinsäuren gelang nach Einführung der Festphasenmethoden, wobei die zu verlängernden Nukleinsäurefragmente an unlösliche Träger fixiert sind. Die Immobilisierung des "wachsenden" Nukleinsäureteils ermöglicht so die jeweilige Reinigung des gewünschten Reaktionsproduktes durch einfaches Waschen der festen Phase. Lösliche Reaktionspartner werden mit der Festphase in Kontakt gebracht und nach Beendigung der

Reaktion durch Waschen entfernt.

R. Frank et al., Nucl. Acids Res. 11, 4365 - 4377 (1983) beschrieben den Einsatz von Cellulose in Form von Papierblättchen ("disks") als feste Phase für die chemische Synthese von kurzen DNA-Fragmenten. Papier, Cellulose und generell alle Polysaccharide haben aber den grundsätzlichen Nachteil, daß alle nicht für die Fixierung der Nukleinsäure oder Nukleinsäurefragmente benötigten reaktiven Gruppen, wie Hydroxylreste, blockiert werden müssen, damit keine Störung der Synthesezyklen erfolgt. Eine vollständige Blockierung aller nicht als Nukleotidanker benötigten reaktiven Gruppen von als Trägermaterialien verwendeten Polysacchariden ist jedoch unmöglich, um so mehr als bei vielmaliger Wiederholung der Reaktionszyklen sich oft die makroskopische Struktur solcher festen quellfähigen Träger, z. B. durch Ausbildung von Rissen, ändert und so vorher unzugängliche reaktive Gruppen dann für Reaktanden zugänglich werden und zu Störungen des Reaktionsablaufs oder zu Nebenreaktionen führen können. Speziell bei Verwendung von Papier als Trägermaterial für die chemische Synthese von Nukleinsäuren kann so eine große Zahl von Fehlsequenzen auftreten. Die mangelnde mechanische Stabilität von Papier bei längerer Chemikalienbeanspruchung bringt außerdem Schwierigkeiten in der maschinellen Synthese mit sich, weswegen bisher Sequenzen von mehr als 20 - 25 Basen so nicht dargestellt werden.

Bei quellbaren Trägern können je nach Reaktionsmedium durch Quellung bzw. Entquellung des Materials beträchtliche Wasch-und Reaktionszeiten auftreten. Außerdem kann es zu Diffusionsproblemen kommen. Wegen dieser Nachteile werden üblicherweise nicht quellbare Träger für die chemische Umsetzung insbesondere die Synthese von Nukleinsäuren oder Nukleinsäurefragmenten eingesetzt. Ein gebräuchliches Material hierfür ist Silicagel.

Silicagel ist ein oft eingesetzter unlöslicher Träger für den chemischen Aufbau von Nukleinsäuren. Dieses Material besitzt eine breite Verteilung verschiedener Porengrößen und eine unregelmäßige Porenstruktur. Die chemische Synthese von Polynukleotiden an modifizierten anorganischen Polymeren, wie Silicagel, ist in EP-A-0035719 beschrieben.

In EP-A-0090789 ist ein Verfahren zur chemischen Synthese von DNA mittels der Phosphittriestermethode an einer Festphase beschrieben. Als Festphase wird bevorzugt ein sogenanntes Controlled Pore Glass mit einem Porendurchmesser von ca. 5-2500 Å beschrieben.

Für die Synthese doppelsträngiger DNA unter Verwendung sowohl chemischer als auch enzymatischer Reaktionsschritte wurden von Z. Hostomsky & J. Smrt in Nucl. Acids Res. 18, 241- 244 (1987) Fractosil $1000^R$, ein Silicagel mit einer Porengröße von 1000 Å und Sephacryl-$500^R$, ein durch dreidimensionale Vernetzung von linearen Dextran-Ketten mit N,N'-Methylen-bis(acrylamid) erhältliches, hydrophiles, gelbildendes, d. h. quellbares Material auf ihre Eignung als Trägermaterialien miteinander verglichen. Es wurde dabei festgestellt, daß das nicht quellbare Silicagel im Gegensatz zu dem gelbildenden Träger die enzymatischen Reaktionen, Ligierung bzw. Freisetzung der fertigen Nukleinsäure vom Trägermaterial teilweise bzw. vollständig inhibierte.

Nach wie vor bestand deshalb bis heute Bedarf an Trägermaterial für den kombinierten Einsatz chemischer und enzymatischer Methoden zur Umsetzung von Nukleinsäuren oder Nukleinsäurefragmenten an unlöslichem, polymeren Trägermaterial, das für chemische Reaktionen die Nachteile quellbarer Träger vermeidet und vorteilhaft für enzymatische Reaktionen eingesetzt werden kann.

Die Aufgabe der Erfindung war es, geeignete Träger für die chemische oder/und enzymatische Umsetzung von Nukleinsäuren oder Nukleinsäurefragmenten an Festphasen bereitzustellen, die die vorstehenden Anforderungen erfüllen.

Gelöst wird diese Aufgabe durch die in den Ansprüchen charakterisierte Erfindung.

Der erfindungsgemäße Träger zur chemischen und enzymatischen Umsetzung von Nukleinsäuren oder Nukleinsäurefragmenten besteht aus einem unlöslichen, nicht oder nicht wesentlich quellbaren, porösen Polymeren, an das die zur Reaktion zu bringenden Nukleinsäuren oder Teile von Nukleinsäuren eventuell mit in der Nukleinsäurechemie üblichen Schutzgruppen versehen, fixiert sind, wobei die Poren des Polymeren im wesentlichen eine definierte Größe innerhalb eines bestimmten Bereiches aufweisen. Die Poren müssen einerseits so groß sein, daß Enzyme im wesentlichen ungehinderten Zugang zu den zur Reaktion zu bringenden fixierten Nukleinsäuren oder Teilen von Nukleinsäuren haben. Andererseits müssen die Poren so klein sein, daß der Träger eine große Oberfläche aufweist, die viele Reaktionsstellen anbietet, um so mit möglichst wenig Trägermaterial möglichst viele Nukleinsäure-Moleküle umsetzen zu können. In diesem Sinne haben sich Träger als bevorzugt erwiesen, die Poren einer definierten Größe zwischen 1400 und 10000 Å besitzen. Bevorzugt sind solche Polymere, deren Poren im wesentlichen definierte Größen zwischen 2000 und 5000 Å, insbesondere zwischen mehr als 2500 und 5000 Å aufweisen, wobei unter "definiert" eine maximale Abweichung von ± 10 % vom angegebenen Wert verstanden wird.

Überraschenderweise hat sich gezeigt, daß solche Polymere, die diese Porengrößenbedingungen erfüllen und die von ihrer Zusammensetzung her gegenüber den für die chemische und enzymatische

Umsetzung von Nukleinsäuren erforderlichen Agenzien resistent sind, sich gut für sowohl chemische als auch enzymatische Reaktionen, wie z. B. solche für die Synthese von Nukleinsäuren eignen, die manuell oder automatisch durchgeführt werden können. Insbesondere wenn es sich bei dem Trägerpolymeren um ein Material handelt, das wässrige Lösungen, insbesondere Pufferlösungen, als Medien für Enzymreaktionen nicht aus den Hohlräumen ausschließt, laufen enzymatische Reaktionen, wie Ligierungen mit Ligasen oder Abspaltungen von Nukleinsäuren oder Nukleinsäurefragmenten, z. B. mit Restriktionsenzymen, schnell und vollständig ab.

Als bevorzugte Trägerpolymere werden anorganische Materialien, insbesondere solche, die im wesentlichen aus Silicium-und Sauerstoffatomen aufgebaut sind, eingesetzt.

Ganz besonders geeignet hat sich für chemische und insbesondere auch für enzymatische Umsetzungen von Nukleinsäuren oder Nukleinsäurefragmenten Glas mit einer kontrollierten Porengröße von etwa 3000 Å erwiesen.

Die Art des polymeren Trägermaterials ist ausschlaggebend für die vorteilhafte Einsatzmöglichkeit des erfindungsgemäßen Trägers für den kombinierten Einsatz chemischer und enzymatischer Methoden zur Umsetzung von Nukleinsäuren oder Nukleinsäurefragmenten. So ist es z. B. nicht nötig, nach Abschluß einer chemischen Synthese von Nukleinsäureketten diese von der hierfür verwendeten festen Phase abzutrennen und für die enzymatische Kettenverlängerung ein anderes Trägermaterial zu wählen. Verlustreiche Abtrenn- und Ankopplungsreaktionen können mit dem erfindungsgemäßen Träger vermieden werden. Ganz besonders für enzymatische Umsetzungen ist der erfindungsgemäße Träger hervorragend geeignet.

Die immobilisierten Nukleinsäuren oder Nukleinsäurefragmente können auf die unterschiedlichste Art und Weise an das Trägermaterial fixiert sein. Besonders vorteilhaft ist es jedoch, wenn der Träger zur Festphasensynthese von Nukleinsäuren die eventuell mit in der Nukleinsäurechemie üblichen Schutzgruppen versehenen Nukleinsäurefragmente kovalent an das unlösliche, nicht quellbare, poröse Polymere gebunden enthält. Für die chemische Synthese von Nukleinsäuren geht man dabei in bekannter Weise meist von eventuell geschützten Monomeren entsprechender Nukleinsäuren aus, es können aber auch Oligomere eingesetzt werden. Bei der enzymatischen Umsetzung sind oft größere Nukleinsäurefragmente an das polymere Trägermaterial gebunden. Die enzymatische Umsetzung von kleinen Nukleinsäurefragmenten ist aber natürlich oft genauso möglich.

In einer bevorzugten Ausführungsform des erfindungsgemäßen Trägers sind die Nukleinsäurefragmente über einen Spacer an das Polymer gebunden. Als Spacer kommen eine Vielzahl von Substanzen in Betracht, die von dem Fachmann je nach den zur Frage stehenden Umsetzungen ausgewählt werden können. Gesichtspunkte für eine solche Wahl sind insbesondere die Stabilität gegenüber den jeweils gewählten Reaktionsbedingungen bei sowohl den chemischen als auch den enzymatischen Umsetzungen. Ganz besonders vorteilhaft hat es sich innerhalb der Methoden der chemischen Nukleinsäuresynthese für die heutzutage vorherrschende Phosphitmethode erwiesen, wenn die zur Reaktion zu bringende eventuell mit in der Nukleinsäurechemie üblichen Schutzgruppen versehene Nukleinsäure oder der Teil einer Nukleinsäure mit einem Aminopropylsilyl-Spacer

$$(-\overset{|}{\underset{|}{Si}}-(CH_2)_3-NH-)$$

an das Trägermaterial gebunden ist. Speziell Träger der allgemeinen Formel I

$$\text{Polymer}-O-\overset{OR}{\underset{OR'}{\overset{|}{\underset{|}{Si}}}}-CH_2-CH_2-CH_2-NH-\overset{O}{\overset{\|}{C}}-CH_2-CH_2-\overset{O}{\overset{\|}{C}}-O-\text{Nukl.} \qquad (I)$$

in der

| | |
|---|---|
| Polymer | ein Glas mit einer kontrollierten Porengröße zwischen mehr als 2500 und 5000 Å, bevorzugt von etwa 3000 Å, |
| R und R′, | die gleich oder verschieden sein können, $C_1$-$C_5$-Alkyl oder Polymer, und |
| Nukl. | eine eventuell mit in der Nukleinsäurechemie üblichen Schutzgruppen versehenes Fragment einer Nukleinsäure |

bedeuten,

haben sich für die kombinierte chemische und enzymatische Synthese von Nukleinsäuren hervorragend bewährt. Insbesondere enzymatische Umsetzungen können hieran vorteilhaft durchgeführt werden.

Das Fragment einer Nukleinsäure in der Bedeutung von Nukl. kann sowohl ein einzelnes Nukleotid als auch ein Oligomer aus 2 - 100 Monomereinheiten oder ein größeres Polymer mit mehreren hundert Monomereinheiten sein.

Als Fragment einer Nukleinsäure in der Definition von Nukl. haben sich für enzymatische Umsetzungen besonders Oligonukleotide aus 6 - 30 Nukleotiden bewährt. Ganz besonders bevorzugt sind solche Oligonukleotide aus 15 - 25 Nukleotiden.

Analog dem von T. Atkinson und M. Smith bei M. J. Gait (Ed.) in "Oligonucleotide synthesis - a practical approach", IRL Press, Oxford, Washington D.C. (1984), S. 45 - 49 beschriebenen Verfahren kann ein solcher ganz besonders bevorzugter Träger durch Aminopropylierung von controlled pore glass 3000 Å (CPG 3000, Serva, Heidelberg, Bundesrepublik Deutschland) und Umsetzung mit dem entsprechenden am 3′-Ende durch p-nitrophenylsuccinyl substituierten eventuell durch in der Nukleinsäurechemie üblichen Schutzgruppen versehenen Nukleinsäurefragment hergestellt werden. Es können so Beladungsdichten von etwa 1 µmol bis 30 µmol Nukleinsäure oder Nukleinsäurefragment pro Gramm polymerem Trägermaterial hergestellt werden. Besonders bevorzugt sind Träger mit einer Beladungsdichte von 5 bis 11 µmol/g.

Ein weiterer Gegenstand der Erfindung ist ein Verfahren zur Synthese von Nukleinsäure nach einer Festphasenmethode, wobei als feste Phase ein unlösliches, nicht quellbares, poröses Polymeres eingesetzt wird, dessen Poren im wesentlichen eine definierte Größe zwischen 1400 und 10 000 Å besitzen. Vorzugsweise haben die Poren eine kontrollierte Größe zwischen 2000 und 5000 Å, insbesondere zwischen mehr als 2500 und 5000 Å.

Unter Festphasenmethoden werden hier alle diejenigen Verfahren zur Synthese von Nukleinsäuren verstanden, die heterogen ablaufen, d. h. wo ein Nukleinsäureteil, sei es ein Monomer, wie ein eventuell mit in der Nukleinsäurechemie üblichen Schutzgruppen versehenes Nukleosid oder Nukleotid, oder ein aus mehreren Monomereinheiten bestehendes eventuell ebenfalls geschütztes Nukleinsäurefragment an ein unlösliches Trägermaterial fixiert wird, mittels chemischer und enzymatischer Reaktionen oder mittels enzymatischer Reaktionen alleine um eine oder mehrere Monomereinheiten verlängert und nach Abschluß der Synthese die fertige Nukleinsäure gegebenenfalls von der festen Phase abgelöst wird.

Für die chemische Synthese von Nukleinsäuren sind das Phosphodiester-, Phosphotriester- und das Phosphitverfahren üblich, wobei sich gerade die beiden letzten Methoden und hiervon insbesondere die Phosphitmethode für die Festphasensynthese von Nukleinsäuren als vorteilhaft erwiesen haben.

Während sich mit chemischen Verfahren am besten Oligonukleotide aus etwa bis zu 60 Monomereinheiten synthetisieren lassen, können diese mit enzymatischen Methoden zu Nukleinsäuren aus mehreren hundert Monomereinheiten verlängert werden, wie dies bereits in der Beschreibung des Standes der Technik wiedergegeben ist. Übliche Enzyme sind z. B. Kinasen, Polymerasen und Ligasen. Auch zur Abspaltung der an einer Festphase fertig synthetisierten Nukleinsäure von der Trägermatrix können Enzyme eingesetzt werden, z. B. Restriktionsendonukleasen.

Bisher war eine effiziente, eventuell automatisierbare Synthese von Nukleinsäuren, die chemische und enzymatische Verfahrensschritte beinhaltet, nur möglich, wenn im Falle des Einsatzes quellbarer Festphasen die Nachteile mangelnder mechanischer Stabilität, langer Wasch- und Reaktionszeiten sowie eventueller Nebenreaktionen oder im Falle des Einsatzes nicht quellbarer Festphasen eventuelle Inhibierungen eingesetzter Enzyme und damit unvollständige Reaktionen in Kauf genommen wurden. Zur Vermeidung einiger dieser Nachteile konnten die chemischen Syntheseschritte auf einem nicht quellbaren Trägermaterial und die enzymatischen Syntheseschritte auf einem quellbaren Material durchgeführt werden. Dies bedeutete jedoch, daß während der Synthese das Trägermaterial gewechselt werden mußte und verlustreiche Abtrenn- und Ankopplungsreaktionen durchgeführt werden mußten.

Das erfindungsgemäße Verfahren mit den Schritten:
Fixierung eventuell mit Schutzgruppen versehener Nukleinsäurefragmente an ein unlösliches, nicht quellbares, poröses Polymer, Verlängerung der immobilisierten Nukleinsäurefragmente durch weitere Nukleinsäurefragmente und gegebenenfalls Abspaltung fertiger Nukleinsäuren vom unlöslichen Polymer weist diese Nachteile nicht auf. Es ist in sehr vorteilhafter Weise für den kombinierten chemischen und enzymatischen Nukleinsäureaufbau geeignet. Ein Wechsel des Trägermaterials bei Kombination chemischer und enzymatischer Syntheseschritte ist nicht nötig. Ganz besonders ist das Verfahren für die enzymatische Nukleinsäuresynthese geeignet.

Erfindungsgemäß wird als Trägermaterial ein im wesentlichen aus Silicium- und Sauerstoffatomen bestehendes Polymer bevorzugt, das sich durch seine große mechanische Stabilität und durch seine hohe permanente Porosität und geringe Quellungsporosität besonders auszeichnet. Dies gilt ganz besonders für Glas einer definierten Porengröße zwischen 1400 und 10000 Å. Insbesondere Glas einer kontrollierten

Porengröße von etwa 2000 - 5000 Å, vorzugsweise zwischen mehr als 2500 und 5000 Å und ganz besonders solches einer kontrollierten Porengröße von etwa 3000 Å zeigt hervorragende Eigenschaften, die es als nicht quellbares, poröses Material für eventuell automatisierbare Festphasenverfahren empfehlen. Besonders hervorzuheben ist hierbei die Eignung zur Immobiliserung von Nukleinsäuren oder Nukleinsäurefragmenten und die Eignung solcher festphasengebundener Substanzen als Enzymsubstrate zu wirken.

Nukleinsäurefragmente können auf die unterschiedlichste Art und Weise an das Trägermaterial fixiert werden. Besonders vorteilhaft ist es jedoch, wenn die Nukleinsäurefragmente, die gegebenenfalls mit Schutzgruppen versehen sein können, kovalent an das unlösliche, nicht quellbare Polymere gebunden werden, vorzugsweise über einen Spacer, wobei als Spacer eine Vielzahl von Substanzen in Betracht kommen, die von dem Fachmann je nach den zur Frage stehenden Umsetzungsbedingungen gewählt werden können. Ganz besonders vorteilhaft hat es sich erwiesen, wenn die Nukleinsäurefragmente, die gegebenenfalls durch eine in der Nukleinsäurechemie übliche Schutzgruppe geschützt sein können, mit einem Aminopropylsilyl-Spacer

$$(-\overset{|}{\underset{|}{Si}}-(CH_2)_3-NH-)$$

an das Trägermaterial gebunden werden, z. B. mittels $(C_2H_5O)_3Si$-O-$(CH_2)_3$-$NH_2$. Insoweit sind die vorstehend beschriebenen Träger der allgemeinen Formel I besonders geeignet für das erfindungsgemäße Verfahren.

Eine Inhibierung von Enzymen wird hierbei nicht beobachtet. Dies gilt insbesondere für Polynukleotidkinase, DNA-Polymerase sowie RNA- und DNA-Ligase und Restriktionsendonukleasen. Das erf indungsgemäße Verfahren eignet sich deshalb ganz besonders für enzymkatalysierte Umsetzungen immobilisierter Nukleinsäuren oder Nukleinsäurefragmente. Beispiele solcher enzymatischen Umsetzungen sind die enzymatische Phosphorylierung von Oligonukleotidfragmenten, wie z. B. Oligodeoxythymidylat mit $T_4$-Polynukleotid-Kinase,
das Anligieren von DNA-Fragmenten an Oligonukleotide, wie z. B. Oligodeoxythymidylat mit Hilfe von DNA-Ligase in Gegenwart eines Gegenstrangfragments,
Kettenverlängerung durch einsträngiges Anknüpfen von DNA-Fragmenten mit 3′-Riboterminus an ein immobilisiertes Oligonukleotid, wie z. B. Oligodeoxythymidylat, mit Hilfe von RNA-Ligase,
Replikation eines immobilisierten RNA-DNA-Hybridstranges durch Klenow-DNA-Polymerase in Gegenwart eines universell einsetzbaren Starteroligonukleotids, wie z. B. Oligodeoxyadenylat oder
Abschneiden eines immobilisierten Oligonukleotid-Doppelstranges durch den Einsatz von Restriktionsenzymen.

Ein weiterer Gegenstand der Erfindung ist die Verwendung eines unlöslichen, nicht quellbaren, porösen Polymeren, dessen Poren im wesentlichen eine definierte Größe zwischen 1400 und 10000 Å haben, als Trägermaterial für die Immobilisierung von Nukleinsäuren oder Nukleinsäurefragmente, insbesondere dann, wenn solche trägerfixierten Verbindungen zur enzymatischen oder chemischen und enzymatischen Umsetzung von Nukleinsäuren oder Nukleinsäurefragmenten an einer Festphase eingesetzt werden. Solche Polymere eignen sich auch zum Einsatz für Syntheseautomaten zum Aufbau von Nukleinsäuren, da sie sich durch hohe mechanische Stabilität und dauerhafte Belastbarkeit mit wechselnden Reagenzien und Lösungsmitteln auszeichnen. Ganz besonders geeignet sind die vorgenannten Polymere jedoch als Trägermaterial für enzymatische Umsetzungen, da diese daran ungehindert ablaufen.

Bevorzugt sind anorganische Polymere, insbesondere solche, die im wesentlichen aus Silicium- und Sauerstoffatomen aufgebaut sind. Vorteilhafterweise besitzen solche Polymere Poren definierter Größe zwischen 2000 und 5000 Å, insbesondere zwischen mehr als 2500 und 5000 Å. Ganz besonders vorteilhaft hat sich Glas mit einer kontrollierten Porengröße von etwa 3000 Å erwiesen.

Solche weitporigen, nicht quellbaren Polymere eignen sich für die chemische Synthese, weil sie leicht auswaschbar sind, so daß Reagenzienüberschüsse und Lösungsmittel gut entfernt werden können. Außerdem erlaubt das erfindungsgemäße Trägermaterial sehr hohe, nahezu quantitative Ausbeute bei den chemischen Kondensationsschritten.

Als besonders vorteilhaft erweist sich die erfindungsgemäße Verwendung weitporigen, nicht quellbaren Polymers für enzymatische Reaktionen zum Aufbau oder zur Abkopplung von Nukleinsäuren vom Trägermaterial. Enzyme werden durch das Material nicht inhibiert und akzeptieren die damit immobilisierten Nukleinsäuren oder Nukleinsäurefragmente als Substrate.

Im folgenden wird die Erfindung durch Beispiele näher erläutert. Diese sollen jedoch keine Einschränkung des Erfindungsgegenstandes bedeuten.

In den Beispielen verwendete Abkürzungen:

| | |
|---|---|
| Tris | Tris(hydroxymethyl)-aminomethan |
| DTT | Dithiothreitol |
| ATP | Adenosin-5′-triphosphat |
| EDTA | Ethylendiamin-tetraessigsäure |
| Cpm | Counts pro Minute |
| HEPES | 4-(2-Hydroxyethyl)-1-piperazin-ethansulfonsäure |
| DMSO | Dimethylsulfoxid |
| PEG | Polyethylenglykol |
| TE | Tris/EDTA-Puffer |
| BSA | Rinderserum-Albumin |
| DMTrdT | mit Dimethoxytritylgruppe in 5′-Position substituiertes Deoxythymidin |
| MSNT | 1-(Mesitylensulphonyl)-3-nitro-1,2,4-triazol |
| dNTP | Desoxynukleosidtriphosphat |
| TEA | Triethylamin |

**Beispiel 1**

**Synthese immobilisierter $(dT)_{20}$ Oligonukleotide**

**A) Synthese immobilisierten monomeren Deoxythymidins**

**a)Trägermaterial Controlled Pore Glass (CPG) (Serva, Heidelberg, Bundesrepublik Deutschland)**

**Aminopropylierung**

Die Aminopropylierung des Trägermaterials erfolgte analog der Literaturstelle Matteucci und Caruthers, J. Amer. Chem. Soc 103, 3185 - 3191 (1981).

Ansatz: 5 g CPG 1400 bzw. CPG 3000
2,6 ml 3-Aminopropyl-triethoxysilan
3 ml Trimethylchlorsilan

CPG 1400 bzw. CPG 3000 wird durch Umsetzung mit 3-Aminopropyl-triethoxysilan in 50 ml Toluol funktionalisiert. Die Reaktionsmischung wird 12 h bei Raumtemperatur geschüttelt und anschließend 18 h unter Rückfluß gekocht. Der Träger wird abgesaugt und dreimal mit je 20 ml Toluol, dreimal mit je 20 ml Methanol und zweimal mit je 20 ml 50 %iger wässriger Methanol-Lösung gewaschen. Das CPG wird dann über Nacht in wässriger Methanol-Lösung geschüttelt. Die flüssige Phase wird abgetrennt und das CPG zweimal mit je 20 ml Methanol gewaschen. Anschließend wird es zunächst an der Luft, anschließend im Vakuum getrocknet.

Nicht umgesetzte Hydroxylgruppen des Glases werden durch Reaktion mit Trimethylchlorsilan in 10 ml abs. Pyridin blockiert. Die Suspension wird über Nacht geschüttelt, anschließend abgesaugt und das Glas fünfmal mit je 20 ml Methanol und dreimal mit je 20 ml Diethylether gewaschen. Nach Lufttrocknung erfolgt die vollständige Trocknung des aminopropylierten CPG im Vakuum.

**5′-O-DMTr-3′-O-Succinyl-nucleosid**

Analog dem Verfahren von Matteucci und Caruthers, J. Amer. Chem. Soc. 103, 3185 - 3191 (1981) werden
2,5 mmol DMTrdT
2,0 mmol (200 mg) Bernsteinsäureanhydrid
300 mg 4-N,N-Dimethylaminopyridin (DMAP)
5 ml Pyridin, abs.
umgesetzt. 5′-O-DMTr-geschütztes Deoxythymidin wird in Pyridin aufgelöst, zweimal absolutiert und mit DMAP sowie Bernsteinsäureanhydrid versetzt. Der Ansatz wird 12 Stunden bei Raumtemperatur gerührt und dann dünnschichtchromatographisch geprüft. Bei ausreichendem Umsatzgrad wird das Pyridin entfernt und der Rückstand in 30 ml Dichlormethan aufgelöst. Die Dichlormethanlösung wird gegen eisgekühlte 10 %ige wässrige Zitronensäure ausgeschüttelt und die organische Phase anschließend zweimal mit je 15 ml Wasser gewaschen. Nach Trocknen der Dichlormethanschicht über Natriumsulfat wird die Lösung am

Rotationsverdampfer konzentriert und in 250 ml Petrolether ausgefällt. Der Niederschlag wird abgesaugt und noch zweimal mit je 20 ml Petrolether nachgewaschen. Die Ausbeute beträgt 90 % der Theorie.

**Darstellung des p-Nitrophenylesters**

Analog dem Verfahren von Matteucci und Caruthers, J. Amer. Chem. Soc. 103, 3185 - 3191 (1981) werden
1 mmol 3′-O-succinyliertes Nucleosid (wie vorstehend beschrieben hergestellt)
1 mmol (207 mg) Dicyclohexylcarbodiimid (DCC)
1 mmol (140 mg) 4-Nitrophenol
3 ml Dioxan, abs.
0,2 ml Pyridin, abs.
umgesetzt. 5′-O-Dimethoxytrityl-3′-O-succinyl-deoxythymidin wird in der pyridinhaltigen Dioxanlösung gelöst und das DCC zugefügt. Aus der anfangs klaren Lösung fällt innerhalb kurzer Zeit Dicyclohexylharnstoff aus. Nach zwei Stunden Reaktionsdauer wird die Reaktionsmischung dünnschichtchromatographisch geprüft. Dicyclohexylharnstoff wird abfiltriert und das Filtrat sofort mit dem vorbereiteten Trägermaterial umgesetzt.

**Umsetzung der Aktivester mit dem aminopropylierten Träger**

Analog Matteucci und Caruthers, J. Amer. Chem. Soc. 103, 3185 - 3191 (1981) werden
1 mmol 5′-O-DMTr-3′-O-(4-nitrophenyl)succinyldeoxythymidin
2,5 g Trägermaterial
8 ml Dimethylformamid, wasserfrei
1 ml Triethylamin, wasserfrei
1 ml Acetanhydrid
5 ml Pyridin, wasserfrei
50 mg 4-N,N-Dimethylaminopyridin (DMAP)
umgesetzt. Aminopropyliertes CPG wird in Dimethylformamid suspendiert. Die Lösung des Nitrophenyl-Aktivesters des Monomeren wird zugesetzt, Triethylamin zugegeben und die Reaktionsmischung vorsichtig geschüttelt. Nach 12 Stunden Reaktionsdauer wird der Träger abgesaugt und mit Dimethylformamid, Methanol und Diethylether gewaschen. Nach dem Trocknen an der Luft wird die Substanz in Vakuum aufbewahrt.

Die Maskierung unumgesetzter Aminogruppen erfolgt durch Umsetzung mit Essigsäureanhydrid in absolutem Pyridin. Als Katalysator wird DMAP zugesetzt. Nach 30 Minuten wird der Träger abgesaugt, mit Methanol gewaschen, an der Luft und im Vakuum getrocknet.

**b) Trägermaterialien Cellulose bzw. Sepharose**

**Beladung der organischen Trägermaterialien mit 3′-O-succinyliertem Nucleosid**

Analog R. Frank et al., Nucl. Acids Res. 11, 4365 - 4377 (1983) werden
2 g Trägermaterial (Whatman Filterplättchen, Nr. 3, Durchmesser 0,9 mm bzw. Sepharose 4B CL in Dioxan)
1 mmol 3′-O-succinyliertes Deoxythymidin
2,08 g (7 mmol) MSNT
0,2 ml 1-Methylimidazol
150 ml absolutes Pyridin
50 ml Pyridin
50 ml Chloroform
50 ml Ether
20 ml Acetanhydrid
1 g 4-N,N-Dimethylaminopyridin (DMAP)
zur Reaktion gebracht.

Das Trägermaterial wird dreimal mit jeweils etwa 20 ml absolutem Pyridin versetzt und absolutiert. Dann wird das 3′-O-succinylierte Nucleosid, MSNT und 1-Methylimidazol zugegeben und der Ansatz 3 Stunden bei Raumtemperatur geschüttelt. Die sich während dieser Zeit schwarz verfärbende Reaktionslösung wird in einer Schlenk'schen Fritte weitgehend vom Träger abgesaugt und das Trägermaterial mit Pyridin, Chloroform und Ether wieder weiß gewaschen und getrocknet. Zur Blockierung noch unumgesetzter Hydroxylgruppen wird der Träger mit 40 ml einer Mischung aus 80 ml Pyridin, 20 ml Acetanhydrid und 1 g DMAP zwei Stunden lang geschüttelt. Danach wird das Material erneut mit Pyridin, Chloroform und Ether gewaschen und getrocknet.

**c) Bestimmung der Trägerbeladung**

Die Monomer-Beladung des Trägers wird durch die spektrophotometrische Quantifizierung der Farbe des Dimethoxytrityl(DMTr)-kations bestimmt: Eine 1 mg Probe wird entnommen, das DMTr-Kation durch Säurebehandlung freigesetzt und die Absorption bei 498 nm bestimmt. Die Desoxyribonucleosid-Beladung wird nach der Formel berechnet:

$$\frac{E_{498}(nm) \times V\ (ml) \times 14{,}3}{\text{Einwaage (mg)}} = x\ \text{umol Nucleotid/g Träger}$$

$$_{498} = 7 \times 10^4\ cm^2\ mmol^{-1}$$

Es werden die folgenden Beladungen ermittelt:

| Träger | Monomer | Beladung (umol/g) |
|---|---|---|
| CPG 1400 | DMTrdT | 15,8 |
| CPG 3000 | DMTrdT | 10,4 |
| Cellulose | DMTrdT | 96,2 |
| Sepharose | DMTrdT | 91,3 |

**B) Synthese von CPG-($dT_{20}$), Cellulose-($dT_{20}$) und Sepharose($dT_{20}$)**

**a)Synthese von CPG-($dT_{20}$)**

Jeweils 20 - 50 mg mit dem unter A) hergestellten Deoxythymidin (3′-Terminus) beladenen CPG-Material werden in eine Stahlsäule oder Kartusche gefüllt, die durch ein Einspannsystem befestigt und mit einem SAM I-Synthesizer (Firma Biosearch) verbunden ist.

| Reagenzien: | |
|---|---|
| Detritylierung: | 3 % Trichloressigsäure in Dichlormethan |
| Kondensation: | DMTrdTp$^{III}$($NR_2$,Me) mit R = isopropyl 350 mg Tetrazol in 10 ml absolutem Acetonitril wasserfreies Acetonitril |
| Oxidation: | 500 mg Jod in 125 ml Tetrahydrofuran, 12,5 ml Wasser und 1 ml Pyridin |
| Capping: | 12,5 ml Acetanhydrid in 12,5 ml TEA, 75 ml Acetonitril und 4 ml 1-Methylimidazol |
| Waschschritte: | wasserfreies Acetonitril |

Die einzelnen Schritte eines Zyklus laufen wie folgt ab:

1. Detritylierung:

Die 3 %ige Trichloressigsäure in Dichlormethan wird zur Abspaltung der Dimethoxytrityl-Gruppe 2 - 3 Minuten lang durch das Trägermaterial gepumpt. Die rotgefärbten Detritylierungslösungen werden in einem Probensammler aufgefangen und können zur Ausbeutebestimmung herangezogen werden.

2. Waschen:
Säurespuren werden durch Waschen mit Acetonitril entfernt.
3. Kondensation:
Von dem aktivierbaren Nucleosid ($DMTrdTp^{III}(NR_2,Me)$ mit R = isopropyl) wird pro Kondensationszyklus 50 mg in einer Konzentration von 50 mg/ml in einem Vorratsgefäß vorgelegt, aus dem während den Kondensationszyklen vermischt mit der Tetrazol-Lösung das Monomere innerhalb einer Minute durch die Säule gepumpt wird. Anschließend wird das reaktionsfähige Monomere zur Verbesserung der Ausbeute 8 Minuten lang über eine Schleife durch die Säule umgepumpt.
4. Waschen:
Überschüssig eingesetzte Reaktanden werden durch mehrere Minuten dauerndes Waschen mit Acetonitril ausgespült.
5. Oxidation:
Durch zweiminütiges Durchpumpen der Jodlösung durch die Säule wird der dreiwertige Phosphor des Phosphorigsäuretriesters zum fünfwertigen Phosphor aufoxidiert.
6. Waschen:
Das Auswaschen der Oxidationslösung erfolgt wiederum durch Waschen mit Acetonitril.
7. Capping (Maskierung unumgesetzter Hydroxylgruppen):
Die Capping-Lösung wird zwei Minuten lang durch das Trägermaterial gepumpt.
8. Waschen:
Die Capping-Lösung wird durch Waschen mit Acetonitril aus dem Reaktionsgemisch entfernt.

Der Ablauf eines Synthesezyklus im SAM-Synthesizer erfordert einen Zeitaufwand von etwa 25 Minuten.

**b) Synthese von Cellulose-($dT_{20}$) und Sepharose-$(dT)_{20}$**

Bei den Cellulose- und Sepharose-Materialien wurden die unter a) genannten Waschschritte auf etwa die doppelte Zeit verlängert (von üblicherweise zwischen 2 und 3 Minuten auf ca. 5 Minuten) Nach der Detritylierung war die Einführung eines zusätzlichen Waschschrittes mit Dichlormethan vor dem Waschen mit Acetonitril erforderlich,um alle noch im Trägermaterial befindlichen Säurespuren vollständig entfernen zu können. (2 Minuten Waschen mit Dichlormethan)

**c) Beladungsdichte**

Die Herstellung von ($dT_{20}$)-Oligonukleotiden an den verschiedenen Trägermaterialien im SAM I-Synthesegerät ergab folgende Beladungen:

| Träger | Endbeladung |
|---|---|
| CPG 1400 | 11,7 umol/g |
| CPG 3000 | 7,8 umol/g |
| Cellulose-F.P. | 25,4 umol/g |
| Sepharose CL | 30,7 umol/g |

Nach Abspaltung der Schutzgruppen (DMTr und Methoxy) verblieb das Oligonucleotid an der festen Phase und wurde nach Entfernung von Reagenzien und organischen Lösungsmitteln bis zur Verwendung für enzymatische Umsetzungen getrocknet und im Kühlschrank aufbewahrt.

**d) Schutzgruppenabspaltung**

**Abspaltung der DMTr-Schutzgruppe:**

Während einer Synthese wird die DMTr-Gruppe mit 2 % Trichloressigsäure in Methylenchlorid während 2 min. bzw. 3 % Dichloressigsäure in Methylenchlorid innerhalb 3 min abgespalten. Soll das Produkt ohne Affinitätschromatographie aufgereinigt werden, so erfolgt die sofortige Detritylierung des Oligonucleotids noch im Syntheseapparat. Nach einer affinitätschromatographischen Aufreinigung erfolgt die Abspaltung der DMTr-Schutzgruppe vom Oligonucleotid durch eine 30minütige Behandlung mit 80 %iger Essigsäure. Nach erfolgter Lyophilisation wird zweimal Wasser zugegeben und erneut lyophilisiert. Die DMTr-Gruppe wird dann mit Ether extrahiert und das Produkt eingedampft.

**Abspaltung der Methoxy-Gruppe am Phosphat**

Die Abspaltung der Methoxygruppe erfolgt durch Behandlung des Trägers mit einer frisch bereiteten Lösung aus Thiophenol : TEA : Dioxan = 1 : 2 : 2 über einen Zeitraum von 45 min. Die flüssige Phase wird anschließend abgetrennt und der Träger dreimal mit 1 ml Methanol und dreimal mit 1 ml Diethylether gewaschen.

**Beispiel 2**

**Enzymatische 5′-Phosphorylierung eines immobilisierten $(dT)_{20}$ Oligonucleotids**

Zu 100 µg CPG3000-($dT_{20}$) aus Beispiel 1 (ca. 500 pmol - 1 nmol 5′-OH-Enden) wird 1 ul Kinase-Puffer (400 mM Tris-Salzsäurepuffer pH 7,6, 100 mM Magnesiumchlorid, 10 mM DTT, 50 % Glycerin) 1 ul -$^{32}$p-ATP sowie 1 ul einer 100 uM kalten ATP-Lösung, 1 ul T4-Polynukleotid-Kinase zugefügt und mit bidestilliertem Wasser auf ein Gesamtvolumen von 10 ul aufgefüllt. Die Reaktionsmischung wird 15 Minuten bei 37 °C inkubiert. Zur quantitativen Phosphorylierung der freien 5′-Hydroxylgruppen wird anschließend 1 ul 1 mM ATP zugegeben und weitere 15 Minuten bei 37 °C reagieren gelassen. Die Beendigung der Reaktion erfolgt durch Zugabe von 1 ul 250 mM EDTA-Lösung/10 ul Reaktionslösung

| Reaktionsansatz: | | |
|---|---|---|
| Stammlösung | Reagenz | Endkonzentration |
| | CPG 3000 -$^{32}$p-ATP | 0,1 uM |
| 400 mM | Tris.HCl pH 7,6 | 40 mM |
| 100 mM | Magnesiumchlorid | 10 mM |
| 10 mM | DTT | 1 mM |
| 50 % | Glycerin | 5 % |
| 4 u/ul | T4-PN-Kinase | 400 u/ml |
| 10 ul | Gesamtvolumen | |

Der Träger wird anschließend zur Entfernung ungebundener Reaktanden und Puffer fünfmal mit jeweils 500 ul 0,1 M Dikaliumhydrogenphosphat-Lösung gewaschen. Die Effizienz der Waschungen wird durch Cerenkov-Messung überprüft. Die am Träger verbleibende Restaktivitätsmenge liegt bei diesem Verfahren zwischen 8 x $10^5$ und 2,4 x $10^6$ cpm.

$^{32}$P zerfällt mit einer Halbwertszeit von 14 Tagen. Durch die Cerenkov-Messung werden die Zerfälle pro Minute (counts per minute, cpm) ermittelt. Dies erfolgt in einem formalen Szintillationszähler. Die Radioaktivitätsmenge zwischen 8 x $10^5$ und 2,4 x $10^6$ cpm läßt einen Schluß auf die Menge des eingebauten radioaktiven Phosphats im am Trägermaterial immobilisierten Oligonucleotid zu. Da anschließend durch Zugabe von "kaltem" ATP vollständig phosphoryliert wird, macht die Radioaktivitätsmenge eine Aussage darüber, ob die Kinase-Reaktion überhaupt stattgefunden hat, oder sei es durch irgendwelche Kontaminationen im Träger oder durch Salze bzw. Unreinheiten des Oligonucleotids inhibiert war. Höhere Radioaktivitätsmengen sind günstig, da das Oligonucleotid über einen längeren Zeitraum sichtbar gemacht werden kann. Die Kinasierung des Träger-Oligonucleotids ist Voraussetzung für alle übrigen enzymkatalysierten Umsetzungen.

Beim Einsatz größerer Trägermengen (500 ug - 1 mg) wird in einem Gesamtvolumen von 40 ul gearbeitet, die anfängliche ATP-Konzentration liegt bei 100 uM, nach 15 Minuten wird 1 ul einer 10 mM ATP-Lösung zugesetzt. Reaktionstemperatur und - dauer bleiben gleich.

**Beispiel 3**

**Enzymatische Verknüpfung von immobilisierten DNA-Fragmenten mit DNA-Ligase**

Das kovalent an CPG 3000 gebundenes, 5′-terminal phosphorylierte Oligothymidylat aus Beispiel 2 wird zunächst in 15 ul Wasser mit Oligoadenylat und einem weiteren nicht immobilisierten Oligonucleotid, welches als 3′-Ende ein Oligothymidylat besitzt, durch dreiminütiges Aufheizen auf 100 °C und anschließendes, langsames Abkühlen auf Raumtemperatur hybridisiert. 2 ul DNA-Ligase-Puffer (760 mM Tris-Salzsäure-Puffer, pH 7,6, 10 mM Magnesiumchlorid, 1 mM ATP), 1 ul 1 mM ATP und 2 ul DNA-Ligase

werden zugegeben und das Reaktionsgemisch 6 - 24 Stunden bei 20 °C inkubiert.

| Reaktionsansatz: | | |
|---|---|---|
| Stammlösung | Reagenz | Endkonzentration |
| | CPG 3000-$(dT)_{20}$p | |
| | Oligo A | |
| | Oligo T | |
| 1,5 mM | ATP | 150 uM |
| 10 mM | Magnesiumchlorid | 1 mM |
| 760 mM | Tris.HCl, pH 7,6 | 76 mM |
| 6 u/ul | DNA-Ligase | 600 u/ml |
| 20 ul | Gesamtvolumen | |

Zur Bestimmung der Ausbeuten wird eine Probe entnommen (2 ul Suspension) durch 30minütige Ammoniak-Behandlung vom Träger abgespalten und lyophilisiert. Anschließende Gelelektrophorese an einem 10 - 20 %, denaturierenden Polyacrylamidgel, 0,4 mm, sowie Ausschneiden der Edukt- und Produktbanden nach der Anfertigung des Autoradiogramms und Gelelution mit 0,5 M Ammoniumacetat, 1 mM EDTA wird durch Cerenkov-Vergleichsmessung der Umsatzgrad ermittelt.

Tabelle

| DNA-Ligase-Reaktionen: | | | |
|---|---|---|---|
| CPG 3000-$(dT)_{20}$-Phosphat | Oligo A | Oligo T | Ausbeute an Ligierungsprodukt |
| 1 mg | $(dA)_{20}$ 1 nmol | $(dT)_{10}$ 1,1 nmol | 47 % |
| 1 mg | $(dA)_{12-18}$ 0,8 nmol | $(dT)_{10}$ 1,1 nmol | 47 % |
| 500 ug | p$(dA)_{25-30}$ 2,3 nmol | $(dT)_{10}$ 5,5 nmol | 69 % |
| 100 ug | $(dA)_{20}$ 1 nmol | $(dCTAGGT_{10})$ 1 nmol | 95 % |

## Beispiel 4

### Enzymatische Verknüpfung von immobilisierten DNA-Fragmenten und Desoxyoligonucleotiden mit ribo-Terminus mit RNA-Ligase

Alle Puffer und Lösungen, die eingesetzt werden, werden zunächst autoklaviert oder steril filtriert.

Zu 100 µg immobilisiertem 5′-terminal phosphorylierten Eicosathymidylat aus Beispiel 2 wird Desoxyoligonucleotid mit 3′-Ribo-Terminus gegeben und lyophilisiert. Anschließend werden 15 ul 100 mM ATP-Lösung, 4 ul 10 mM Spermin, 4 ul 100 mM Magnesiumchloridlösung und 4 ul 100 mM DTT-Lösung dazugegeben und lyophilisiert. 2 ul 500 mM HEPES und 3 ul DM50 werden zugefügt, kurz geschüttelt und dann mit 10 ul 40 %iger Polyethylenglykol-Lösung sowie mit 5 ul RNA-Ligase aufgefüllt. Die Reaktionsmischung wird 48 Stunden bei Raumtemperatur inkubiert.

| Reaktionsansatz: | | |
|---|---|---|
| Stammlösung | Reagenz | Endkonzentration |
| | Träger-$(dT)_{20}$p Oligonucleotid-rA | |
| 100 uM | AtP | 75 mM |
| 10 mM | Spermin | 2 mM |
| 100 mM | Magnesiumchlorid | 20 mM |
| 100 mM | DTT | 20 mM |
| 500 mM | HEPES-Puffer, pH 7,5 | 50 mM |
| 100 % | DMSO | 15 % |
| 40 % | Polyethylenglykol (MW 6000) | 20 % |
| 11 mg/ml | RNA-Ligase | 28 ug/ul |
| 20 ul | Gesamtvolumen | |

| Durchgeführte Umsetzungen: | | | |
|---|---|---|---|
| Ansatz | gelöstes Oligonucleotid | Menge | Ausbeute |
| 1 | $d(T_7A_2)rA$ | 1 nmol | 55 % |
| 2 | $d(T_7A_2)rA$ | 2 nmol | 70 % |
| 3 | $d(T_7A_2)rA$ | 4 nmol | 63 % |
| 4 | $d(T_7A_2)rA$ | 4 nmol | 66 % |
| 5 | $d(T_7A_2)rA$ | 5 nmol | 82 % |
| 6 | d(GATCCA)rA | 5 nmol | 80 % |
| 7 | d(GATCCA)A | 2 nmol | 66 % |
| 8 | $d(N)_{48}rA$ | 500 pmol | 30 % |
| 9 | $d(N)_{48}rA$ | 500 pmol | 40 % |
| 10 | $d(N)_{48}rA$ | 700 pmol | 46 % |

Nach erfolgter Umsetzung wird der Träger dreimal mit jeweils 500 ul TE (10 mM Tris-Salzsäure-Puffer, pH 7,6, 2,5 mM EDTA) gewaschen, eine Probe entnommen, mittels Ammoniakbehandlung vom Träger abgespalten und gelelektrophoretisch an einem 10 - 20 %igen Polyacrylamidgel aufgetrennt. Analog zum Verfahren bei den DNA-Ligasereaktionen aus Beispiel 3 werden die Ausbeuten nach der Gelelution durch Cerenkov-Vergleichsmessungen bestimmt.

Wenn nach der ersten RNA-Ligasereaktion weitere RNA-Ligase-Reaktionen durchgeführt werden sollen, muß zunächst eine quantitative Phosphorylierung der DNA-Termini mit Hydroxylenden erfolgen. Hierfür muß der Träger zur Entfernung von Reagenzien aus der RNA-Ligasereaktion phenolisiert werden:

- Zugabe von 500 ul Phenol, 30 Sekunden mixen, 1 Minute zentrifugieren, Phenolphase abnehmen;
- Zugabe von 500 ul Phenol: Chloroform = 1:1, 30 Sekunden mixen, 1 Minute zentrifugieren, organische Phase abnehmen;
- Zugabe von 500 ul Isoamylalkohol: Chloroform = 1:25, 30 Sekunden mixen, 1 Minute zentrifugieren, organische Phase abnehmen, kurz evaporieren.

Durch eine anschließende erneute Durchführung der Phosphorylierungsreaktion mit T4-Polynucleotid-Kinase liegt die Radioaktivität des Trägers wieder auf 1 - 2 x $10^6$ cpm und die 5′-Termini sind phosphoryliert. Eine weitere, im Anschluß durchgeführte Einzelstrangverknüpfung mit RNA-Ligase gibt am Beispiel der Trägerprobe 10, welche als immobilisierte Oligomere die Oligonucleotide $(dT)_{20}$p und $dT_{20}$-$(dN)_{48}$Ap enthält, folgende Umsetzungen:

| Träger | gelöstes Oligonukleotid | Produkt Ausbeute | Nebenprodukt Ausbeute |
|---|---|---|---|
| $CPG-T_{20}rAd(N)_{48}p$ | $d(N)_{32}rA$ | $CPG-dT_{20}rAd(N)_{48}rAd(N)_{32}$ 19 % | $CPG-dT_{20}rAd(N)_{32}$ 11 % |

Abkürzungen:

$d(N)_{48}rA$ = 5' CGC CAT CAT CAA GAA CGC CTA CAA GAA GGG CGA GTG ATA ACT GCA GCA rA

$d(N)_{32}rA$ = 5' GAG CCA GAC GCC CCT GGT GAC GCT GTT CAA AA rA

## Beispiel 5

### Replikation eines immobilisierten Oligonucleotids mit Hilfe von DNA-Polymerase (Klenow-Fragment)

Das universell einsetzbare Starteroligonucleotid $(dA)_{10-18}$ wird in 16 ul bidestilliertem Wasser und 3 ul Nicktranslation-Puffer (500 mM Tris-Salzsäure-Puffer, pH 7,2, 100 mM Magnesiumsulfat, 1 m MDTT, 500 µg/ml BSA) mit dem immobilisierten Eicosathymidylat aus Beispiel 1 hybridisiert. Dazu heizt man 3 Minuten auf 100 °C auf und läßt dann vorsichtig auf Raumtemperatur abkühlen. Nach Zugabe von 5 ul 10 mM Desoxynucleosidtriphosphat (enthält jeweils 2,5 mM dATP, dGTP, dCTP und dTTP) und 3 ul $-^{32}p$-dATP sowie 3 ul des Klenow-Fragments der DNA-Polymerase wird die Replikation des am Träger immobilisierten Oligonucleotids eingeleitet.

| Reaktionsansatz: | | |
|---|---|---|
| Stammlösung | Reagenz | Endkonzentration |
| | Träger-$(dT)_{20}$-Oligo. | |
| 2 uM | Oligo A $-^{32}p$-ATP | 0,2 uM |
| 10 mM | dNTP | 1,6 uM |
| 500 mM | Tris.HCl pH 7,2 | 50 mM |
| 100 mM | Magnesiumsulfat | 10 mM |
| 1 mM | DTT | 0,1 mM |
| 500 ug/ml | BSA | 50 ug/ml |
| 30 ul | Gesamtvolumen | |

Durchgeführte Umsetzungen:

a) 200 pmol $(dA)_{16}$ werden in Gegenwart von 2 u Klenow-Polymerase nach Hybridisierung an CPG3000-$dT_{20}$-$rA(dA_2T_7)$ aus Beispiel 4 verlängert. Bestimmung des Erfolgs der Umsetzung durch Cerenkov-Vergleichsmessung nach fünfmaligem Waschen mit jeweils 500 µl TE (10 mM Tris-HCl, 0,2 5MM EDTA, pH 8,0):

| | |
|---|---|
| cpm Träger vor der Replikation: | 41 400 |
| cpm Träger nach der Replikation: | 243 850 |
| cpm Träger nach Denaturierung: | 52 570 |
| cpm Überstand nach Denaturierung: | 184 330 |

b) Ca. 1 nmol des Oligoadenosin-Produkts aus dem Überstand nach Denaturierung aus Beispiel 5 a) (0,25 O.D.) als Oligonucleotidprimer und 15 u Klenow-Polymerase werden analog Beispiel 5 a) eingesetzt.

Ermittlung des Erfolgs der Replikation durch Gelelektrophorese. Es werden zwei Proben A und B aufgetragen.

Bahn A): Banden nach Ammoniak-Behandlung einer Trägerprobe
Ergebnis: homologe Reihe ab ca. 14 mer bis 30 mer 20 mer und 30 mer zeigen stärkere Bande
Bahn B) : Banden nach Trägerdenaturierung (nur Überstand)
Ergebnis: homologe Reihe ab ca. 14 mer bis 30 mer

## Beispiel 6

**Einsatz von Restriktionsenzymen zum Abschneiden eines Oligonucleotid-Doppel stranges vom Träger**

Eingesetzt wird CPG3000-$dT_{20}$-rA($dA_2T_7$) nach erfolgter Klenow-Reaktion aus Beispiel 5
Der Ansatz wird in einem Gesamtvolumen von 20 ul durchgeführt. Eine ca. 25 ug Trägerprobe (ein Viertel des Ansatzes aus der DNA-Polymerase-Reaktion) aus Beispiel 5 wird mit 16 ul Wasser versetzt, 2 ul Low-salt-restriktionsendonuclease-Puffer (10 mM Tris-Salzsäure-Puffer, pH 7,5, 10 mM Magnesiumchlorid, 1 m MDTT) und 2 ul EcoRI zugegeben. Der Ansatz wird 1 Stunde bei 37 °C inkubiert.

| Reaktionsansatz: | | |
|---|---|---|
| Stammlösung | Reagenz | Endkonzentration |
| | CPG 3000-$(dT)_{20}$-Oligo. | |
| 10 mM | Tris. Cl pH 7,5 | 1 mM |
| 10 mM | $MgCl_2$ | 1 mM |
| 1 mM | DTT | 0,1 mM |
| 20 ul | Gesamtvolumen | |

Das Ergebnis der Spaltung wird nach Abspaltung der Proben vom Träger und deren Kinasierung durch Gelelektrophorese geprüft, wobei die Probe zunächst geteilt wird. Teil 1 wird hitzedenaturiert, Teil 2 wird sofort durch Ammoniakbehandlung abgespalten (enthält die immobilisierten sowie die durch Hybridisierung gebundenen Sequenzen). Die abgespaltenen Oligonucleotide werden über eine Sephadex G 50-Säule (Pasteur-pipette) mit bidestilliertem $H_2O$, pH 8, als Elutionsmittel entsalzt. Die im Totvolumen eluierten Proben werden lyophilisiert und nach der für Oligonucleotide in Lösung angegebenen Methode phosphoryliert. Zur Überprüfung des Ergebnisses der Spaltung wird ein Polyacrylamidgel eingesetzt.

Man erhält Produkte, die alle kürzer als das eingesetzte 30 mer sind. Es wird keine Inhibierung des Restriktionsenzyms beobachtet.

## Beispiel 7

Anligieren eines DNA-Fragmentes mit Hilfe von DNA-Ligase an ein an einen quellfähigen Träger gebundenes Oligonukleotid

| Reaktionsansätze: | | | | |
|---|---|---|---|---|
| Träger/Menge | Oligo A | $dT_{10}$ | Ausbeute Ligierungsprodukt ges. | $(dT)_{30}$ |
| CPG 3000 500 ug | $p(dA)_{25-30}$ 2,3 nmol | 5,5 nmol | 69 % | 51 % |
| Cellulose Fp 1 mg | $p(dA)_{25-30}$ 2,3 nmol | 5,5 nmol | 68 % | 43 % |
| Sepharose CL4B 1 mg | $p(dA)_{25-30}$ 2,3 nmol | 5,5 nmol | 65 % | 41 % |

Die angegebenen Ausbeuten sind bezogen auf den Umsatz des radioaktiv markierten Oligonucleotids am Träger.

Alle drei verwendeten Trägermaterialien waren bei der Kinasierung mit T4-PN-Kinase und der DNA-Ligasereaktion vergleichbar. Die beiden quellfähigen Träger waren jedoch sehr viel schwieriger von eingesetzten Reaktanden (deutlich sichtbar beim Auswaschen des eingesetzten radioaktiv markierten ATP) zu befreien. Selbst nach ausführlichen Waschschritten waren bei der Gelelektrophorese noch Anteile nicht kovalent gebundenen radioaktiven Phosphats vorhanden.

Bei der DNA-Ligasereaktion waren die Ausbeuten bei allen drei Trägern vergleichbar. Aufgrund der besseren chemischen Synthese an CPG 3000 ist jedoch die Ausbeute am gewünschten Ligationsprodukt $(dT)_{30}$ bei CPG 3000 eindeutig besser.

Cellulose Filterplättchen weisen bei der chemischen Synthese das Problem auf, daß durch Entstehen neuer Hydroxylgruppen durch die mechanische Beanspruchung des Trägermaterials Sequenzen kürzerer Kettenlänge gebildet werden. Dies ist beobachtbar durch Zunahme der Tritylfarbe.

Am problematischsten ist eine chemische Synthese bei Sepharose CL. Da der Träger äußerst quellfähig ist, müssen Waschschritte zwischen den Reaktionsschritten über einen bedeutend längeren Zeitraum durchgeführt werden. Die Automatisierung der Synthese ist nur sehr schwierig durchführbar: Außerdem ist wie bei Cellulose eine Zunahme der Tritylfarbe beobachtbar, so daß auch hier eine homologe Produktreihe, wenngleich mit deutlich stärker auftretendem Endprodukt entsteht.

## Patentansprüche

1. Träger zur enzymatischen oder chemischen und enzymatischen Umsetzung von Nukleinsäuren oder Nukleinsäurefragmenten an einer festen Phase bestehend aus einem unlöslichen, nicht quellbaren, porösen Polymeren, an das ein oder mehrere eventuell mit in der Nukleinsäurechemie üblichen Schutzgruppen versehene Nukleinsäuren oder Nukleinsäurefragmente fixiert sind, dadurch gekennzeichnet, daß die Poren des Polymeren im wesentlichen eine definierte Größe zwischen mehr als 2500 und 5000 Å besitzen.

2. Träger gemäß Anspruch 1, dadurch gekennzeichnet, daß das Polymer im wesentlichen aus Silicium- und Sauerstoffatomen aufgebaut ist.

3. Träger gemäß Anspruch 2, dadurch gekennzeichnet, daß das Polymer ein Glas ist.

4. Träger gemäß einem der Ansprüche 1-3, dadurch gekennzeichnet, daß eventuell mit Schutzgruppen versehene Nukleinsäurefragmente kovalent an das Polymere fixiert sind.

5. Träger gemäß Anspruch 4, dadurch gekennzeichnet, daß eventuell mit Schutzgruppen versehene Nukleinsäurefragmente über Aminopropylsilyl-Spacer

$$(-\overset{|}{\underset{|}{Si}}-(CH_2)_3-NH-)$$

an das Polymer fixiert sind.

6. Verfahren zur enzymatischen oder chemischen und enzymatischen von Nukleinsäuren, wobei eventuell mit Schutzgruppen versehene Nukleinsäuren an einem unlöslichen, nicht quellbaren, porösen Polymer chemisch synthetisiert, die immobilisierten Nukleinsäurefragmente durch weitere Nukleinsäurefragmente enzymatisch verlängert und fertige Nukleinsäuren abschließend gegebenenfalls vom unlöslichen Polymer abgelöst werden, dadurch gekennzeichnet, daß das Polymer eine defininierte Porgengröße zwischen 1400 und 10000 Å besitzt.

7. Verfahren gemäß Anspruch 6, dadurch gekennzeichnet, daß das Polymer Glas einer definierten Porengröße zwischen 2000 und 5000 Å ist.

8. Verwendung eines unlöslichen, nicht quellbaren, porösen Polymeren, dessen Poren im wesentlichen eine definierte Größe zwischen 1400 und 10000 Å besitzen, als Trägermaterial für die enzymatische oder chemische und enzymatische Umsetzung von Nukleinsäuren oder Nukleinsäurefragmenten an fester Phase.

9. Verwendung eines im wesentlichen aus Silicium- und Sauerstoffatomen aufgebauten, unlöslichen, nicht quellbaren, porösen Polymeren, dessen Poren im wesentlichen eine definierte Größe zwischen mehr als 2500 und 5000 Å besitzen, gemäß Anspruch 8.

10. Verwendung von Glas einer kontrollierten Porengröße von etwa 3000 Å gemäß Anspruch 8.

## Claims

1. Carrier for the enzymatic or chemical and enzymatic reaction of nucleic acids or nucleic acid fragments on a solid phase consisting of an insoluble, non-swellable, porous polymer on which are fixed one or more nucleic acids or nucleic acid fragments possibly provided with protective groups usual in nucleic acid chemistry, characterised in that the pores of the polymer essentially possess a definite size of between more than 2500 and 5000 Å.

2. Carrier according to claim 1, characterised in that the polymer is essentially built up of silicon and oxygen atoms.

3. Carrier according to claim 2, characterised in that the polymer is glass.

4. Carrier according to one of claims 1 - 3, characterised in that nucleic acid fragments possibly provided with protective groups are fixed covalently on to the polymer.

5. Carrier according to claim 4, characterised in that nucleic acid fragments possibly provided with protective groups are fixed on to the polymer via aminopropylsilyl spacers

$$(-\overset{|}{\underset{|}{Si}}-(CH_2)_3-NH-).$$

6. Process for the enzymatic or chemical and enzymatic solid phase synthesis of nucleic acids, whereby nucleic acid fragments possibly provided with protective groups are chemically synthesised on an insoluble, non-swellable, porous polymer, the immobilised nucleic acid fragments are enzymatically lengthened by further nucleic acid fragments and final nucleic acids are possibly subsequently removed from the insoluble polymer, characterised in that the polymer possesses a definite pore size of between 1400 and 10,000 Å.

7. Process according to claim 6, characterised in that the polymer is glass with a definite pore size of between 2000 and 5000 Å.

8. Use of an insoluble, non-swellable, porous polymer, the pores of which essentially possess a definite size of between 1400 and 10,000 Å, as carrier material for the enzymatic or chemical and enzymatic reaction of nucleic acids or nucleic acid fragments on a solid phase.

9. Use of an insoluble, non-swellable, porous polymer essentially built up of silicon and oxygen atoms, the pores of which essentially possess a definite size of between more than 2500 and 5000 Å.

10. Use of a glass of a controlled pore size of about 3000 Å according to claim 8.

## Revendications

1. Support pour la réaction enzymatique, ou chimique et enzymatique, d'acides nucléiques ou de fragments d'acides nucléiques, sur une phase solide constituée d'un polymère poreux, insoluble, non susceptible dc gonfler, sur lequel sont fixés un ou plusieurs acides nucléiques ou fragments d'acides nucléiques, éventuellement munis de groupes protecteurs utilisés couramment dans la chimie des acides nucléiques, caractérisé en ce que les pores du polymère présentent essentiellement une taille définie, comprise entre plus de 2500 et 5000 Å.

**2.** Support selon la revendication 1, caractérisé en ce que le polymère est essentiellement constitué d'atomes de silicium et d'atomes d'oxygène.

**3.** Support selon la revendication 2, caractérisé en ce que le polymère est un verre.

**4.** Support selon l'une quelconque des revendications 1.3, caractérisé en ce que les fragments d'acides nucléiques, éventuellement munis de groupes protecteurs, sont fixés sur le polymère par des liaisons covalentes.

**5.** Support selon la revendication 4, caractérisé en ce que les fragments d'acides nucléiques, éventuellement munis de groupes protecteurs, sont fixés sur le polymère par l'intermédiaire de groupe intercalaire aminopropylsilyle ($-Si-(CH_2)3-NH-$).

**6.** Procédé de synthèse enzymatique, ou chimique et enzymatique, d'acides nucléiques, dans lequel des acides nucléiques, éventuellement munis de groupes protecteurs, sont synthétisés chimiquement sur un polymère poreux, insoluble, non susceptible de gonfler, les fragments d'acides nucléiques immobilisés sont prolongés enzymatiquement à l'aide d'autres fragments d'acides nucléiques, et ensuite, les acides nucléiques de longueur définitive sont éventuellement séparés du polymère insoluble, caractérisé en ce que le polymère présente une taille de pores définie comprise entre 1400 et 10 000 Å.

**7.** Procédé selon la revendication 6, caractérisé en ce que le polymère est un verre ayant une taille de pores définie, comprise entre 2000 et 5000 Å.

**8.** Utilisation d'un polymère poreux, insoluble, non susceptible de gonfler, dont les pores présentent essentiellement une taille définie. comprise entre 1400 et 10 000 Å, comme matériau de support pour la réaction enzymatique, ou chimique et enzymatique, d'acides nucléiques ou de fragments d'acides nucléiques sur une phase solide.

**9.** Utilisation d'un polymère poreux, insoluble, non susceptible de gonfler, essentiellement constitué d'atomes de silicium et d'atomes d'oxygène, dont les pores présentent essentiellement une taille définie, comprise entre plus de 2500 et 5000 Å, selon la revendication 8.

**10.** Utilisation d'un verre ayant une taille de pores contrôlée, d'environ 3000 Å, selon la revendication 8.